# EUROPEAN PATENT SPECIFICATION

(11) **EP 2 428 237 B1**
(45) Date of publication and mention of the grant of the patent: **03.04.2013**
(21) Application number: 10176081.7
(22) Date of filing: 09.09.2010
(51) Int. Cl.: A61M 5/142

(54) **Fluid dispensing or feeding device**
Flüssigkeitsausgabe- oder Zufuhrvorrichtung
Dispositif d'alimentation ou de distribution de fluides

(43) Date of publication of application: 14.03.2012
(73) Proprietor: Wiswong Technology Corporation, Kaohsiung 80052 Chines Taipei (CN)
(72) Inventor: Wang, Hong Jen, Kaohsiung 80052 (TW)
(74) Representative: Beck, Michael Rudolf

(56) References cited:
- US-A1- 2009 173 754

## Description

The invention relates to a fluid dispensing or feeding device including a pressurize device for pressurizing a fluid to a patient.

Typical fluid dispensing or feeding devices comprise a pressurizing device for forcing a fluid from a bottle and out through a discharge tube without gravity. However, the typical fluid dispensing or feeding devices are complicated and may not be easily manufactured and assembled.

A fluid dispencing device is disclosed in US 7 584 872 A1. The preamble of claim 1 is based on this document.

The invention is to provide a fluid dispensing or feeding device including a simplified coil for pressurizing a fluid.

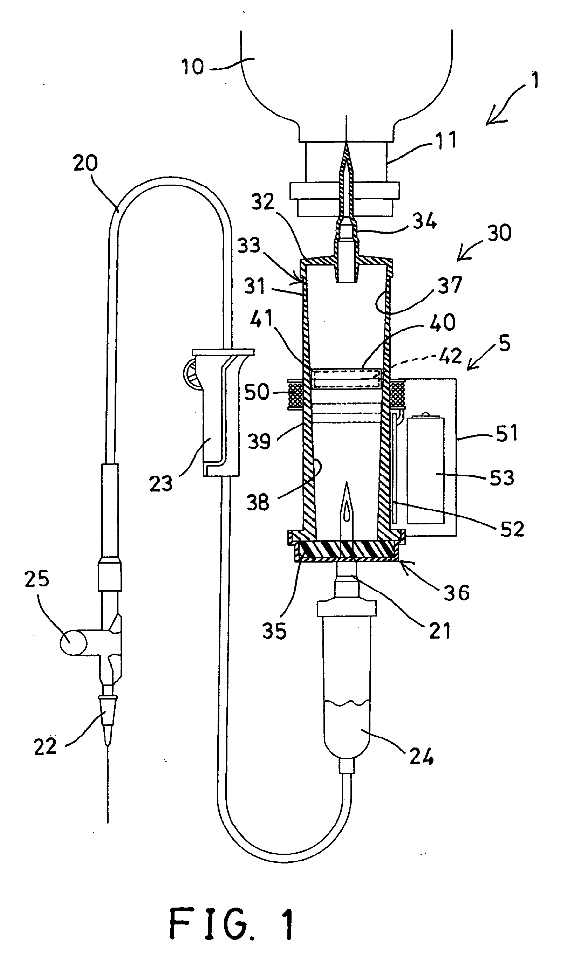

FIG. 1 is a cross sectional view of a fluid feeding device.

Referring to FIG. 1, a fluid dispensing or feeding device 1 comprises a bottle 10 for receiving a fluid, and a discharge tube 20 having a needle 21 for coupling to the bottle 10, and another needle 22 for penetrating into patient's body tissue. A clamp valve 23 is attached onto the tube 20, a drip meter or flow indicating device 24 is positioned in the tube 20 for viewing the drops of fluid passing from the bottle 10 to the tube 20, an air relief valve 25 is attached to the tube 20 for relieving air.

A pressurizing device 30 includes a container 31 having a cap 32 attached to the upper portion 33, and a needle 34 extended from the cap 32 for engaging into the neck portion 11 of the bottle 10, the container 31 includes a plug 35 attached to the lower portion 36 and made of rubber or synthetic materials, and the needle 21 is engaged through the plug 35 and into the container 31, for allowing the fluid to flow out of the fluid container 31 and to flow through the tube 20. A piston 40 is slidably received in a chamber 37 of the container 31 and made of soft or resilient rubber or plastic materials and includes a resilient outer peripheral portion 41 for engaging with an inner peripheral surface 38 of the container 31 which is tapered or inclined from the upper portion 33 or the middle portion 39 toward the middle portion 39 or the lower portion 36 of the container 31 for forming a cone or frustum-shaped surface 38 and for engaging with the outer peripheral portion 41 of the piston 40.

The outer diameter of the piston 40 is greater than the inner diameter of the inner peripheral surface 38 at the middle portion 39 or the lower portion 36 of the container 31 for engaging with the inner peripheral surface 38 and for forcing the fluid toward the plug 35 and into the tube 20 through the needle 21 when the piston 40 is moved toward the plug 35 or away from the cap 32, in addition, the piston 40 is smaller than the inner diameter of the inner peripheral surface 38 at the middle portion 39 or the upper portion 33 of the container 31 for selectively disengaging from the inner peripheral surface 38 of the container 31, and to allow the fluid to flow through the gap or passage between the inner peripheral surface 38 of the container 31 and the outer peripheral portion 41 of the piston 40 when the piston 40 is moved away from the plug 35 or toward the cap 32.

An actuating device 5 includes a coil 50 attached to the middle portion 39 of the container 31, and disposed around the piston 40, and includes one or more magnetic members 42 engaged into the piston 40 for acting with the coil 50 and for allowing the piston 40 to be moved up and down relative to the container 31 and to be moved in a reciprocating action in the container 31 by the action between the coil 50 and the magnetic members 42. A casing 51 is attached to the container 31 for receiving the coil 50 and/or a circuit board 52 and/or one or more batteries 53 which may be coupled to the coil 50 in order to energize and actuate the coil 50.

In operation, when the piston 40 is moved toward the plug 35 or away from the cap 32 by the coil 50 and the magnetic members 42, the fluid contained in the lower portion 36 of the container 31 may be forced to flow toward the plug 35 and to flow into the tube 20 through the needle 21. On the contrary, when the piston 40 is moved away from the plug 35 or toward the cap 32, the fluid contained in the upper portion 33 of the container 31 may flow through the gap or passage between the inner peripheral surface 38 of the container 31 and the outer peripheral portion 41 of the piston 40 and into the lower portion 36 of the container 31.

## Claims

1. A fluid dispensing device comprising a bottle (10) for receiving a fluid, a discharge tube (20), a pressurizing device comprising a container (31) coupled between the bottle (10) and the tube (20), a piston (40) received in the container (31) and having an outer peripheral portion (41) for forcing the fluid into the tube (20),
the container (31) includes an inclined inner peripheral surface (38), the outer peripheral portion (41) of the piston (40) is engageable with the inner peripheral surface (38) of the container (31) for forcing the fluid into the tube (20) when the piston (40) is moved toward the tube (20), and the piston (40) being disengaged from the inner peripheral surface (38) of the container (31) when the piston (40) is moved away from the tube (20), **characterized in that**:
a coil (50) is attached to the container (31) to act and to move the piston (40) in a reciprocating action within the container (31).

2. A fluid dispensing device as claimed in claim 1, wherein the pressurizing device (30) includes at least one magnetic member (42) engaged in the piston (40).

3. A fluid dispensing device as claimed in claim 1 or 2 further comprising at least one battery (53) coupled to the coil (50).

## Patentansprüche

1. Flüssigkeitsausgabevorrichtung, umfassend eine Flasche (10) zur Aufnahme einer Flüssigkeit, einen Ausgabeschlauch (20), eine Druckvorrichtung, die einen Behälter (31) umfasst, der zwischen der Flasche (10) und dem Schlauch (20) angeordnet ist, einen Kolben (40), der in dem Behälter (31) aufgenommen wird und einen äußeren Umfangsbereich (41) aufweist, um die Flüssigkeit in den Schlauch (20) zu drücken,
wobei der Behälter (31) eine abgeschrägte innere Umfangsfläche (38) aufweist, der äußere Umfangsbereich (41) des Kolbens mit der inneren Umfangsfläche (38) des Behälters (31) zusammenwirken kann, um die Flüssigkeit in den Schlauch (20) zu drücken, wenn der Kolben (40) in Richtung des Schlauchs (20) bewegt wird, und sich der Kolben (40) von der inneren Umfangsfläche (38) des Behälters (31) löst, wenn der Kolben (40) vom Schlauch (20) weg bewegt wird, **dadurch gekennzeichnet, dass**
eine Magnetspule (50) an dem Behälter (31) befestigt ist, um eine Hubbewegung des Kolbens (40) innerhalb des Behälters (31) zu bewirken.

2. Flüssigkeitsausgabevorrichtung nach Anspruch 1, wobei die Druckvorrichtung (30) mindestens ein Magnetelement (42) aufweist, das in den Kolben (40) eingesetzt ist.

3. Flüssigkeitsausgabevorrichtung nach Anspruch 1 oder 2, die weiterhin mindestens eine Batterie (53) umfasst, die mit der Magnetspule (50) verbunden ist.

## Revendications

1. Dispositif de distribution de fluide comprenant une bouteille (10) pour recevoir un fluide, un tube d'évacuation (20), un dispositif de pressurisation comprenant un conteneur (31) couplé entre la bouteille (10) et le tube (20), un piston (40) logé dans le conteneur (31) et ayant une portion périphérique extérieure (41) pour pousser le fluide dans le tube (20),
le conteneur (31) contient une surface périphérique intérieure inclinée (38), la portion périphérique extérieure (41) du piston (40) peut être mise en prise avec la surface périphérique intérieure (38) du conteneur (31) pour pousser le fluide dans le tube (20) lorsque le piston (40) est déplacé vers le tube (20) et le piston (40) étant dégagé de la surface périphérique intérieure (38) du conteneur (31) lorsque le piston est éloigné du tube (20),
**caractérisé en ce qu'**une bobine (50) est attachée au conteneur (31) pour agir et pour déplacer le piston (40) dans une action réciproque à l'intérieur du conteneur (31).

2. Dispositif de distribution de fluide selon la revendication 1 dans lequel le dispositif de pressurisation (30) contient au moins un élément magnétique (42) en prise dans le piston (40).

3. Dispositif de distribution de fluide selon la revendication 1 ou 2 comprenant de plus au moins une pile (53) couplée à la bobine (50).
